# EUROPÄISCHE PATENTANMELDUNG

(11) **EP 0 770 597 A1**
(43) Veröffentlichungstag der Anmeldung: **02.05.1997**
(21) Anmeldenummer: 96116468.8
(22) Anmeldetag: 15.10.1996
(51) Int. Cl.: C07C 253/30, C07C 255/19

(54) **Verfahren zur Herstellung von Cyanessigsäurealkylestern**

(30) Priorität: 23.10.1995 DE 19539293
(71) Anmelder: BASF AKTIENGESELLSCHAFT, 67056 Ludwigshafen (DE)
(72) Erfinder: Grund, Clemens, Dr., 68199 Mannheim (DE); Holderbaum, Martin, Dr., 67065 Ludwigshafen (DE); Reichelt, Helmut, Dr., 67435 Neustadt (DE); Beckmann, Stefan, Dr., 67098 Bad Dürkheim (DE)

(57) **Zusammenfassung**

Verfahren zur Herstellung von Cyanessigsäurealkylestern der allgemeinen Formel I 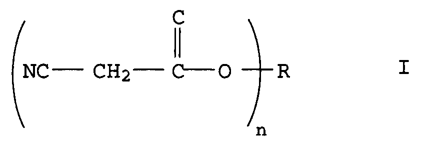 wobei R einen n-wertigen aliphatischen Rest mit 6 bis 20 C-Atomen, dessen Kohlenstoffgerüst durch ein bis drei Sauerstoffatome in Etherfunktion unterbrochen sein kann, und n eine Zahl von 1 bis 6 bedeutet, durch Umsetzung von Cyanessigsäure in wäßrigem Medium mit einem Alkohol R(OH)ₙ, wobei man die Veresterung in Gegenwart eines inerten Schleppmittels durchführt und während der Umsetzung Wasser und Schleppmittel abdestilliert.

## Beschreibung

Die vorliegende Erfindung betrifft ein Verfahren zur Herstellung von Cyanessigsäureestern der allgemeinen Formel I 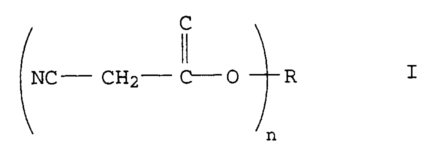 wobei R einen n-wertigen aliphatischen Rest mit 6 bis 20 C-Atomen, dessen Kohlenstoffgerüst durch ein bis drei Sauerstoffatome in Etherfunktion unterbrochen sein kann, und n eine Zahl von 1 bis 6 bedeutet, durch Umsetzung von Cyanessigsäure in wäßrigem Medium mit einem Alkohol R(OH)ₙ.

Cyanessigsäure wird im allgemeinen durch Umsetzung von Chloressigsäure mit Cyanidsalzen hergestellt. Bei dieser Umsetzung fallen wäßrige Rohproduktlösungen an, die im allgemeinen einen Cyanessigsäureanteil von 20 bis 80 Gew.-% bezogen auf die Gesamtmenge der Rohproduktlösung enthalten. Es wurden daher Verfahren zur Veresterung der Cyanessigsäure gesucht, die ohne aufwendige Reinigungsschritte eine Veresterung von Cyanessigsäure in wäßrigem Medium ermöglichen.

In der DE-A 4227505 wird ein Verfahren zur Herstellung von Cyanessigsäure-C₄-C₁₀-alkylestern durch Umsetzung von Cyanessigsäure mit C₄-C₁₀-Alkoholen beschrieben, wobei Cyanessigsäure in wäßrigem Medium mit einem 5 bis 30-fachen molaren Überschuß eines C₄-C₁₀-Alkohols umgesetzt wird und dabei das C₄-C₁₀-Alkohol/Wasser-Azeotrop abdestilliert wird.

Insbesondere bei der Veresterung mit höheren Alkoholen weisen die so erhaltenen Produkte jedoch oft Verfärbungen auf und enthalten unerwünschte Nebenprodukte wie Malonsäuredialkylester. Nachteilig ist zudem die hohe Menge an Alkohol, die zur azeotropen Veresterung benötigt wird.

Aufgabe der vorliegenden Erfindung war es daher, ein Verfahren zur Herstellung von Cyanessigsäureestern I aus wäßrigem Medium zu finden, welches diese Nachteile vermeidet und zu saubereren Produkten führt.

Demgemäß wurde ein Verfahren zur Herstellung von Cyanessigsäurealkylestern der allgemeinen Formel I gefunden 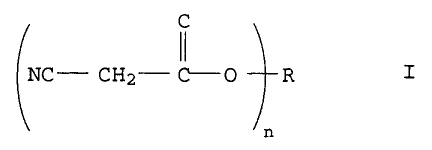 wobei R einen n-wertigen aliphatischen Rest mit 6 bis 20 C-Atomen, dessen Kohlenstoffgerüst durch ein bis drei Sauerstoffatome in Etherfunktion unterbrochen sein kann, und n eine Zahl von 1 bis 6 bedeutet, durch Umsetzung von Cyanessigsäure in wäßrigem Medium mit einem Alkohol R(OH)ₙ, dadurch gekennzeichnet, daß man die Veresterung in Gegenwart eines inerten Schleppmittels durchführt und während der Umsetzung Wasser und Schleppmittel bei atmosphärischem oder vermindertem Druck abdestilliert.

Im erfindungsgemäßen Verfahren wird die Veresterung von Cyanessigsäure in wäßrigem Medium vorgenommen. Dabei geht man in der Regel von einer wäßrigen Cyanessigsäurelösung aus, die einen Gehalt an Cyanessigsäure von 20-80 Gew.-%, vorzugsweise von 60-70 Gew.-%, jeweils bezogen auf das Gewicht der Lösung, enthält.

Besonders vorteilhaft ist die Umsetzung eines wäßrigen Reaktionsgemisches aus Halogenessigsäure und Alkalicyanid in situ mit dem entsprechenden Alkohol, d.h. ohne vorherige Reinigung der entstandenen Cyanessigsäure.

Die eingesetzten Alkohole R(OH)ₙ enthalten als aliphatischen Rest R beispielsweise verzweigte oder unverzweigte Alkylgruppen mit 6 bis 20 C-Atomen.

Besonders bevorzugt werden einwertige Alkohole eingesetzt, die beispielsweise die Reste Hexyl, 2-Methylpentyl, Heptyl, 2-Methylhexyl, Octyl, Isooctyl, 2-Ethylhexyl, Nonyl, 2-Methylnonyl, Isononyl, 2-Methyloctyl, Decyl, Isodecyl, 2-Methylnonyl, Undecyl, Isoundecyl, Dodecyl, Isododecyl, Tridecyl, Isotridecyl, Tetradecyl, Pentadecyl, Hexadecyl, Heptadecyl, Octadecyl, Nonadecyl oder Eicosyl enthalten. (Die Bezeichnungen Isooctyl, Isononyl, Isodecyl und Isotridecyl sind Trivialbezeichnungen und stammen von den nach der Oxosynthese erhaltenen Carbonylverbindungen ab; vgl. dazu Ullmann's Encyclopedia of Industrial Chemistry, 5 th Edition, Vol. A1. Seiten 290-293, sowie Vol. A10, Seiten 284 und 285).

Unter diesen Alkoholen sind diejenigen mit 6 bis 13 C-Atomen bevorzugt.

Besonders bevorzugt wird zur Veresterung 2-Ethylhexanol eingesetzt.

Im Falle n = 2 bis 6 kommen als Beispiele für von Polyolen der Formel R(OH)ₙ abgeleitete n-wertige Reste R insbesondere solche mit 2 bis 12 C-Atomen, welche in ihrem linearen oder verzweigten Kohlenstoffgerüst durch bis zu 3 Sauerstoffatome im Etherfunktion unterbrochen sein können, in Betracht. Einzelne Beispiele hierfür sind:

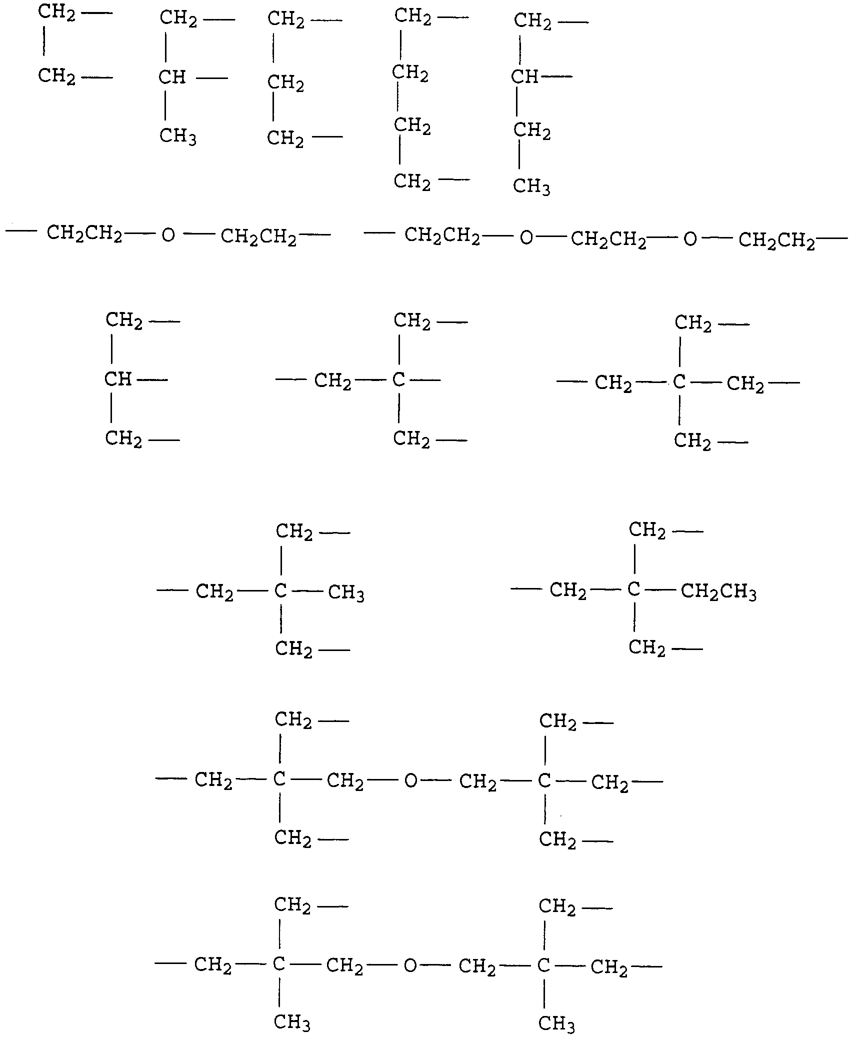

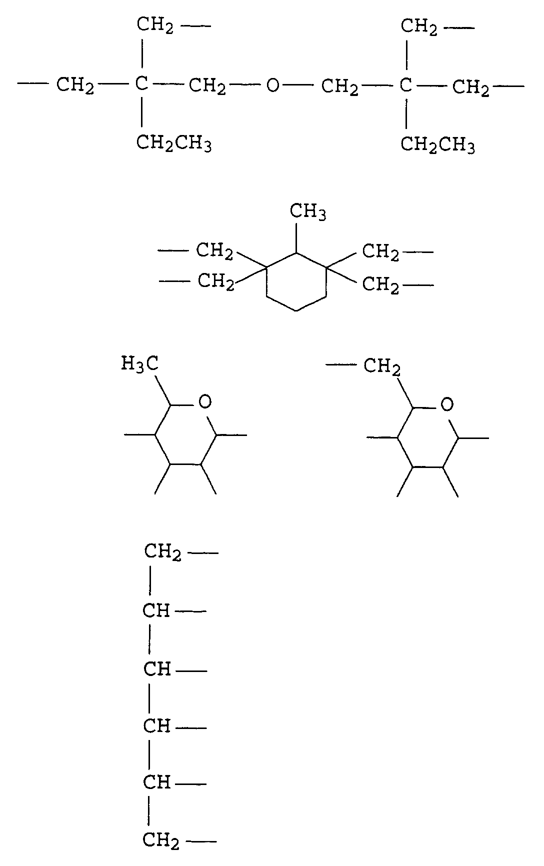

Das bevorzugte Molverhältnis zwischen Cyanessigsäure und Alkoholkomponente hängt von der Wertigkeit des Alkohols ab. Im allgemeinen werden je Mol freier Hydroxylgruppen 0,5 bis 2 Mol Cyanessigsäure eingesetzt, bevorzugt 0,8 bis 1,2 Mol, besonders bevorzugt ca.1 Mol.

Das erfindungsgemäße Verfahren kann bei Normaldruck oder vorteilhaft bei vermindertem Druck durchgeführt werden, wobei ein Druck zwischen 200 und 400 mbar besonders günstig ist.

Die Umsetzungstemperatur hängt wesentlich vom Reaktionsdruck ab, da durch den Druck der Siedepunkt des Gemisches aus Wasser und Schleppmittel vorgegeben ist. Im allgemeinen beträgt die Reaktionstemperatur 50 bis 150°C, bevorzugt 70 bis 130°C, besonders bevorzugt 90 bis 110°C.

Als Schleppmittel können verschiedene inerte Lösungsmittel dienen. Ihre Wahl hängt z.B. von der Verfügbarkeit und vom Siedepunkt ab. Das Azeotrop aus Wasser und Schleppmittel sollte einen niedrigeren Siedepunkt als der eingesetzte Alkohol haben. Als inerte Schleppmittel kommen beispielsweise Toluol, Xylol, Heptan, Cyclohexan oder Methylcyclohexan in Betracht. Besonders bevorzugt wird Toluol eingesetzt.

In einer bevorzugten Ausführungsform wird das Schleppmittel nach dem Abdestillieren vom Wasser getrennt und wieder in das Reaktionsgefäß zurückgeführt. Auf diese Weise ist nur eine relativ geringe Menge Schleppmittel erforderlich. Vorzugsweise wird dem Reaktionsgemisch zwischen 30 und 300 Gew.-% des Schleppmittels, bezogen auf die im Reaktionsgemisch vorhandene Menge Wasser, zugegeben, besonders bevorzugt zwischen 50 und 200 Gew.-%.

Vorteilhaft für die erfindungsgemäße Umsetzung ist der Zusatz eines Katalysators. Als Katalysatoren sind basische Verbindungen wie Carbonate, besonders aber Säuren geeignet.

Geeignete Säuren sind insbesondere starke bis mittelstarke anorganische oder organische Säuren, wie Schwefelsäure, Salzsäure, Phosphorsäure, Benzolsulfonsäure, o- oder p-Toluolsulfonsäure, Methansulfonsäure, Trifluormethansulfonsäure, Trifluoressigsäure oder Mono-, Di- oder Trichloressigsäure. Die Verwendung von Schwefelsäure oder p-Toluolsulfonsäure ist bevorzugt, wobei insbesondere die Verwendung von Schwefelsäure hervorzuheben ist.

Unter katalytischen Mengen werden üblicherweise
0,001 bis 0,01 mol Säure, bezogen auf 1 mol Cyanessigsäure verstanden, wobei von den Mineralsäuren auch größere Mengen, z.B. 0,01 bis 0,1 mol eingesetzt werden können.

Das erfindungsgemäße Verfahren kann sowohl kontinuierlich als auch diskontinuierlich durchgeführt werden.

Das Ende der Umsetzung kann leicht dadurch erkannt werden, daß bei der Destillation kein Wasser mehr übergeht. In einer bevorzugten Ausführungsform der Erfindung wird dann der Druck weiter erniedrigt, z.B. auf 10 bis 50 mbar, und dabei der Rest des Schleppmittels abdestilliert. Anschließend wird abgekühlt, Wasser zugegeben, das Gemisch neutralisiert und die organische Phase von der wäßrigen Phase abgetrennt.

Alternativ kann das Schleppmittel auch nach der Phasentrennung bei erniedrigtem Druck abdestilliert werden.

Das erfindungsgemäße Verfahren führt zu Cyanessigsäurealkylestern von hoher Reinheit und sehr geringer Färbung. Besonders vorteilhaft ist der geringe Anteil von Malonsäuredialkylester im Produkt.

Die erfindungsgemäß hergestellten Cyanessigsäurealkylester sind wichtige Vorprodukte für Lichtschutzmittel.

### Beispiel

912 g (7,5 mol) 70 gew.-%ige wäßrige Cyanessigsäurelösung wurde mit 350 ml Toluol und 974 g (7,5 mol) 2-Ethylhexanol sowie 30 ml 96 gew.-%iger Schwefelsäure vermischt. Das Reaktionsgefäß wurde bis zu einem Druck von 300 mbar evakuiert, und anschließend wurde bei einer Innentemperatur von 100°C das Toluol/Wasser-Gemisch abdestilliert, wobei das abgeschiedene Toluol wieder in das Reaktiongsgefäß zurückgeführt wurde. Nach vollständiger Veresterung wurde bei 25 mbar das Toluol vollständig abdestilliert, dann auf 40°C abgekühlt und 280 ml Wasser zugegeben. Durch Zugabe von 50 gew.-%iger NaOH wurde ein pH-Wert von 7 eingestellt und die organische Phase abgetrennt.
Ausbeute: 88 %

## Patentansprüche

1. Verfahren zur Herstellung von Cyanessigsäurealkylestern der allgemeinen Formel I 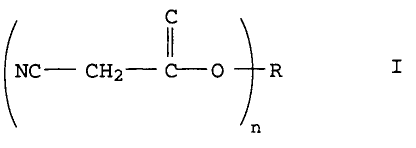 wobei R einen n-wertigen aliphatischen Rest mit 6 bis 20 C-Atomen, dessen Kohlenstoffgerüst durch ein bis drei Sauerstoffatome in Etherfunktion unterbrochen sein kann, und n eine Zahl von 1 bis 6 bedeutet, durch Umsetzung von Cyanessigsäure in wäßrigem Medium mit einem Alkohol R(OH)ₙ, dadurch gekennzeichnet, daß man die Veresterung in Gegenwart eines inerten Schleppmittels durchführt und während der Umsetzung Wasser und Schleppmittel bei atmosphärischem oder vermindertem Druck abdestilliert.

2. Verfahren nach Anspruch 1, dadurch gekennzeichnet, daß n =1 ist.

3. Verfahren nach Anspruch 1 oder 2, dadurch gekennzeichnet, daß R ein aliphatischer Rest mit 6 bis 13 C-Atomen ist.

4. Verfahren nach den Ansprüchen 1 bis 3, dadurch gekennzeichnet, daß R ein 2-Ethylhexylrest ist.

5. Verfahren nach den Ansprüchen 1 bis 4, dadurch gekennzeichnet, daß man die Umsetzung bei einer Temperatur zwischen 70 und 130°C durchführt.

6. Verfahren nach den Ansprüchen 1 bis 5, dadurch gekennzeichnet, daß man die Umsetzung bei einem Druck von 200 bis 400 mbar durchführt.

7. Verfahren nach den Ansprüchen 1 bis 6, dadurch gekennzeichnet, daß man als inertes Schleppmittel Toluol, Xylol, Heptan, Cyclohexan oder Methylcyclohexan einsetzt.

8. Verfahren nach Anspruch 7, dadurch gekennzeichnet, daß man als inertes Schleppmittel Toluol einsetzt.
